(19) [European Patent Office logo] Europäisches Patentamt / European Patent Office / Office européen des brevets

(11) **EP 3 824 815 B1**

(12) **EUROPEAN PATENT SPECIFICATION**

(45) Date of publication and mention
of the grant of the patent:
**05.11.2025   Bulletin 2025/45**

(21) Application number: **18927196.8**

(22) Date of filing: **19.07.2018**

(51) International Patent Classification (IPC):
*A61N 5/10* *(2006.01)*      *A61B 90/18* *(2016.01)*
*A61B 5/11* *(2006.01)*      *A61B 90/10* *(2016.01)*
*A61B 5/00* *(2006.01)*

(52) Cooperative Patent Classification (CPC):
**A61N 5/1049; A61B 5/1114; A61B 5/4887;**
**A61B 90/18;** A61B 2090/101; A61N 2005/1051

(86) International application number:
**PCT/CN2018/096348**

(87) International publication number:
**WO 2020/014934 (23.01.2020 Gazette 2020/04)**

(54) **TUMOR POSITIONING METHOD AND DEVICE**

VERFAHREN UND VORRICHTUNG ZUR TUMORPOSITIONIERUNG

PROCÉDÉ ET DISPOSITIF DE POSITIONNEMENT DE TUMEUR

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**26.05.2021   Bulletin 2021/21**

(73) Proprietors:
• **Our United Corporation**
**Jingkai District**
**Xi'an City**
**Shaanxi 710018 (CN)**
• **Shenzhen our new Medical Technologies**
**Development Co., Ltd.**
**Shenzhen 518057 (CN)**

(72) Inventors:
• **GOU, Tianchang**
**Xi'an, Shaanxi 710016 (CN)**
• **YAN, Hao**
**Xi'an, Shaanxi 710016 (CN)**

(74) Representative: **Cristinelli, Luca**
**Jacobacci & Partners S.p.A.**
**Corso Emilia, 8**
**10152 Torino (IT)**

(56) References cited:
CN-A- 102 697 561      CN-A- 105 324 155
CN-A- 106 730 422      CN-A- 106 955 122
US-A1- 2008 146 905

EP 3 824 815 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to the field of medical instruments, and in particular, to a method and an apparatus of positioning a tumor.

BACKGROUND

At present, radiation therapy is a method of treating tumors using radioactive rays, which may cause complete necrosis or apoptosis of cancer cells, and is one of the main means for treating malignant tumors. During radiation therapy, it is necessary to position the patient's tumor at the position of isocenter, i.e., an intersection of radioactive rays, so that the radioactive rays pass through the tumors to kill the tumor tissues. If there is a deviation in the position of the tumor, it will not only cause the tumor cells not to be effectively killed, increase the risk of secondary cancer, but also cause damage to healthy parts. As a result, one of the key technologies of radiation therapy is to maintain an accurate position of the tumor during treatment.

At present, in the prior art, in order to accurately position the tumor at the isocenter during the radiation therapy of the tumor, it is first necessary for the patient to be given a computed tomography (CT) to obtain the CT image to determine the position, size, shape, and surrounding tissues of the tumor. And then the patient's head is fixed using a treatment head frame during the treatment to position the tumor at the isocenter of the radiotherapy device.

In the method of positioning using of the treatment head frame, four studs disposed in the treatment head frame need to be fixed on the skull of the patient's head through the human skin, and the movement of the tumor is avoided during the treatment by fixing the position of the patient's head. After the treatment is finished, the studs are removed. This method is called an invasive position, and it will cause greater damage to the patient, although the position accuracy thereof is high.

In addition, compared with the above-mentioned invasive position, the related art also provides a non-invasive position method. Generally, in the non-invasive position method, the patient's head is fixed on the treatment couch through a head positioning mask or the like. Compared with the above invasive position method, the non-invasive position method has a problem of instability, and the patient's head may still be moved.

Based on the above situation, the inventors believe that a more optimized method of positioning the tumor is needed to eliminate the effect of the positional change of the patient's head on the result of the tumor position.

CN102697561A discloses a tumor positioning system. The tumor positioning system includes positioning marks for positioning a patient's tumor, a bracket for fixing the positioning marks, an image reconstruction unit for generating a DDR image, a photographing unit for photographing a DR image of a patient's tumor area, a matching unit for performing matching calculation between the DR image and the DDR image, an acquisition unit for acquiring positioning parameters, and a positioning unit for relocating the tumor.

CN106955122A discloses a three-dimensional head detection method based on a single camera module in radiotherapy. Before the radiotherapy, a three-dimensional image of the head is obtained through CT scan results of a patient; during radiotherapy, a face image is obtained in real time; a relative distance and an inclination angle between the current face and the camera module are calculated through the three-dimensional image of the head and the face image; a center of a camera sensor chip of the camera module is used as an original point, and a three-dimensional coordinate point of the head is constructed in real time.

SUMMARY

The embodiments of the present invention provide a method and an apparatus of positioning a tumor, which can eliminate the effect of the positional change of the patient's head on the result of the tumor position, and improve the accuracy of the tumor position.

In order to achieve the above objective, the embodiments of the present invention use the following technical solutions.

In a first aspect, the embodiments of the present invention provide a method of positioning a tumor, and the method of positioning the tumor according to the invention is defined by the appended claim 1.

In a second aspect, the embodiments of the present invention provide an apparatus of positioning a tumor, and the apparatus of positioning the tumor according to the invention is defined by the appended claim 6.

In a third aspect, the embodiments of the present invention provide a computer storage medium, and the computer storage medium according to the invention is defined by the appended claim 7.

In a fourth aspect, the embodiments of the present invention provide a computer program product including instructions, and the computer program product including the instructions is defined by the appended claim 8.

Further preferred embodiments are defined in the dependent claims.

## BRIEF DESCRIPTION OF THE DRAWINGS

In order to more clearly illustrate the embodiments of the present invention or the technical solutions in the prior art, the drawings used in the description of the embodiments or the prior art will be briefly described below. Obviously, the drawings in the following description are only some embodiments of the present invention, and a person of ordinary skill in the art can obtain other drawings according to these drawings without paying any creative effort.

FIG. 1 is a schematic diagram showing a structure of a head positioning mask in the prior art;

FIG. 2 is a flow diagram of a method of positioning a tumor, according to embodiments of the present invention;

FIG. 3 is a schematic diagram showing movement of a patient's head;

FIG. 4 is another schematic diagram showing movement of a patient's head;

FIG. 5 is a schematic diagram of establishing a coordinate system corresponding to a treatment couch at a preset pivot as an origin O of a space coordinate system, according to embodiments of the present invention;

FIG. 6 is a flow diagram of another method of positioning a tumor, according to embodiments of the present invention;

FIG. 7 is a schematic diagram showing a structure of an apparatus of positioning a tumor, according to embodiments of the present invention;

FIG. 8 is a schematic diagram showing a structure of another apparatus of positioning a tumor, according to embodiments of the present invention; and

FIG. 9 is a schematic diagram showing a structure of yet another apparatus of positioning a tumor, according to embodiments of the present invention.

## DETAILED DESCRIPTION

The embodiments of the present invention will be described in the following with reference to the accompanying drawings. The present invention is applied to positioning a tumor in a patient's head.

First, the technical terms involved in the embodiments of the present invention are introduced.

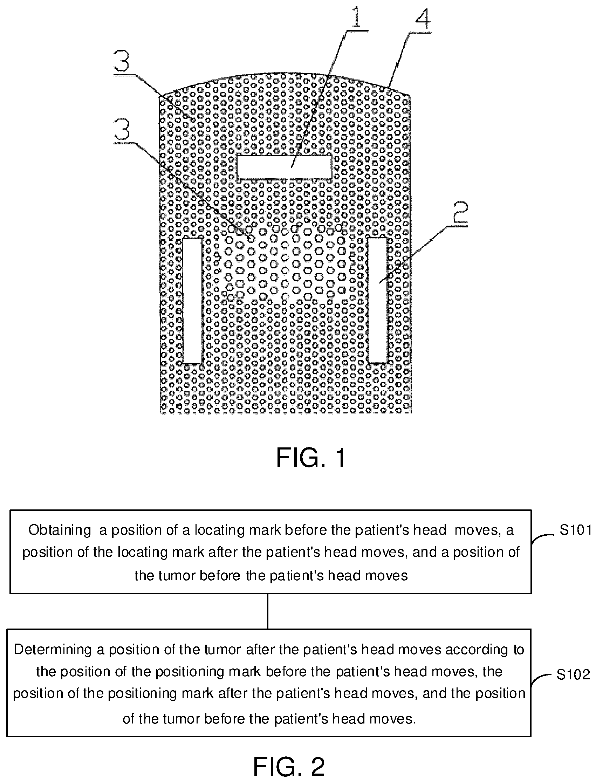

A positioning mask, a new functional medical material with memory function, is made of a high-molecular polymer low-temperature thermoplastic plate, which is widely used for auxiliary position and fixation for the tumor patient in the radiation therapy, reducing unnecessary damage to normal tissues caused by the movement of the patient's body position, and improving the absorption of rays by local lesions. Specifically, according to different fixed positions, positioning masks may be classified into head positioning masks, chest positioning masks, abdominal positioning masks, and body positioning masks. FIG. 1 is a schematic diagram showing a structure of a head positioning mask. The head positioning mask includes a low-temperature thermoplastic plate body 4. A position, corresponding to a nose, in an upper middle portion of the low temperature thermoplastic plate body is provided with a through hole 1, and both sides of the low temperature thermoplastic plate body are provided with a mounting groove, a reinforcing rib 2 is fixed in the mounting groove, and meshes 3 are distributed on the low temperature thermoplastic plate body other than the through hole 1 and the reinforcing rib 2. When the head positioning mask is used, the patient should first be made to lie on the treatment couch, the head positioning mask is placed over the patient's face, and then the patient's head is fixed to the treatment couch through thermoforming of the head positioning mask.

A positioning dental tray, a functional medical material, is used for auxiliary position and fixation for the tumor patient in the radiation therapy. During the radiation therapy, the patient bites the positioning dental tray, so that the position of the patient's head relative to the treatment device is relatively fixed.

The full name of IGRT is image guided radiation therapy. The IGRT is a four-dimensional radiation therapy technology. This positioning method is to scan a 3D image of the entire patient's head by ray irradiation, thereby realizing positioning the tumor.

The inventive concept of the present invention will be described below.

In embodiments of the present invention, in view of accurately positioning the tumor in the process of radiation therapy in the prior art, the patient's head is usually fixed, thereby eliminating the effect of the positional change of the patient's head on the accuracy of the tumor position. However, when the patient's head is fixed, especially when the patient's head is fixed through the means of non-invasive position, there will still be a positional change. For example, when a head positioning mask is used for fixation, the patient's head often rotates around a contact point of the head and the treatment couch. When a positioning dental tray is used for fixation, the patient's head often rotates around an axis of the dental tray. In this case, the position of the tumor will be affected. For this problem, the internal structure image of the patient's entire head is scanned through ray irradiation in the IGRT technology, thereby realizing positioning the tumor. However, this positioning method will cause additional radiation damage to the patient, and the IGRT device is also expensive, so the treatment cost is high.

Further, based on the characteristic that the head of the human body only performs rigid movement, the inventors of the present invention conceive that the overall movement of the patient's head can be known by collecting the positional change situation of a positioning mark on the body surface of the patient's head. And then it is also possible to know the positional change situation of the tumor, thereby achieving precise positioning of the tumor.

Based on the above inventive concept, embodiments of the invention provide a method of positioning a tumor, which may eliminate the effect of the positional change of the patient's head on the result of the tumor position, and improve the accuracy of the tumor position. The following embodiments 1 to 4 are provided for reference.

Embodiment 1

Embodiment of the present invention provides a method of positioning a tumor. As shown in FIG. 2, the method includes the following steps.

In S101, a position of a positioning mark before the patient's head moves, a position of the positioning mark after the patient's head moves, and a position of the tumor before the patient's head moves are obtained.

The positioning mark is disposed at a preset position of a body surface of the patient's head.

For example, the positioning mark in the embodiment of the present invention may be an optical positioning mark, such as an infrared positioning ball, which may be attached to the patient's nasal tip, junction of the ear and the cheek, or the like. Through an optical tracking system, such as an infrared tracking system, the motion of the optical positioning mark may be tracked in time, and then the position of the positioning mark before and after the patient's head moves are obtained.

In addition, the position of the tumor before the patient's head moves may be obtained by calculating a relative position of the tumor in the patient's head through a computed tomography (CT) image, thereby obtaining positional information such as position coordinates of the tumor.

In S102, a position of the tumor after the patient's head moves is determined according to the position of the positioning mark before the patient's head moves, the position of the positioning mark after the patient's head moves, and the position of the tumor before the patient's head moves.

Specifically, since the head of the human body is a whole, and it can only perform rigid movement, the movement situation of the patient's head may be obtained after the positional change of at least one positioning mark on the body surface of the head is obtained. Then, the position of the tumor after the patient's head moves may be obtained after the position of the tumor before the patient's head moves is obtained.

In one implementation manner, S102 may specifically include S102a and S102b.

In S102a, a motion type of the patient's head is determined according to the position of the positioning mark before the patient's head moves, and the position of the positioning mark after the patient's head moves. The motion type at least includes one or two of translation and rotation around a preset pivot.

The S102a may specifically include: determining an amount of movement of the positioning mark according to the position of the positioning mark before the patient's head moves, and the position of the positioning mark after the patient's head moves; determining the motion type of the patient's head according to the amount of movement of the positioning mark.

Specifically, when radiation therapy is performed on the tumor in the patient's head, the patient's head is usually fixed to the treatment couch through the positioning mask or the positioning dental tray. In this case, the patient's head may rotate around the pivot. In rare cases, the patient's head may also move horizontally.

For example, a translation vector of each positioning mark may be calculated according to three-dimensional coordinates of at least two positioning marks in three-dimensional space before and after the movement. If the translation vector of each mark is the same or approximately the same, the motion type of the patient's head is determined to be the translation.

When the translational motion of patient's head is excluded, the motion type of the patient's head is determined according to the patient's fixation mode. For example, if the mask is used for fixation, the motion mode of the patient's head is to rotate around the preset pivot at a back of the patient's head. If the dental tray is used for fixation, the motion mode of the patient's head is to rotate around the preset pivot at the dental tray. Of course, it is also possible to continue to determine which pivot the patient's head is rotated around according to a geometric transformation relationship of at least three positioning marks in three-dimensional space.

Therefore, the positioning marks in the embodiments of the present invention may specifically include a plurality of positioning marks, and then S102a specifically includes: determining amounts of movement of the positioning marks according to the positions of the plurality of positioning marks before the patient's head move, and the positions of the plurality of positioning marks after the patient's head moves; determining the motion type of the patient's head according to the amounts of movement of the positioning marks.

In S102b, a position of the tumor after the patient's head moves is determined according to the motion type of the patient's head and the position of the tumor before the patient's head moves.

After the motion type of the patient's head is determined, the position of the tumor needs to be calculated based on different motion types that the patient's head performs.

In one implementation manner, in a case where it is determined that the motion type of the patient's head is the translation, S102b specifically includes: determining the position of the tumor after the patient's head moves according to amounts of movement of the plurality of positioning marks and the position of the tumor before the patient's head moves.

For example, positions of at least two positioning marks before the patient's head moves, and positions of the at least two positioning marks after the patient's head moves are obtained. The at least two positioning marks are disposed at different preset positions of the body surface of the patient's head respectively. A translation vector of a first positioning mark and a translation vector of a second positioning mark are calculated. It is determined whether there is the translation in the patient's head according to the translation vector of the first positioning mark and the translation vector of the second positioning mark. In a case where it is determined that there is a translational movement in the patient's head, a translation vector of the patient's head is calculated, and the coordinates of the coordinate point of the tumor in the space coordinate system after the patient's head moves are calculated according to the translational vector of the patient's head and the coordinate values of the coordinate point of the tumor in the space coordinate system before the patient's head moves.

In one implementation manner, in a case where it is determined that the motion type of the patient's head is the rotation around a preset pivot, S102b specifically includes the following steps.

In S102b-1, whether the position of the tumor before the patient's head moves coincides with the preset pivot.

In S102b-2, in a case where it is determined that the position of the tumor before the patient's head moves coincides with the preset pivot, the position of the tumor after the patient's head moves is still the position of the tumor before the patient's head moves.

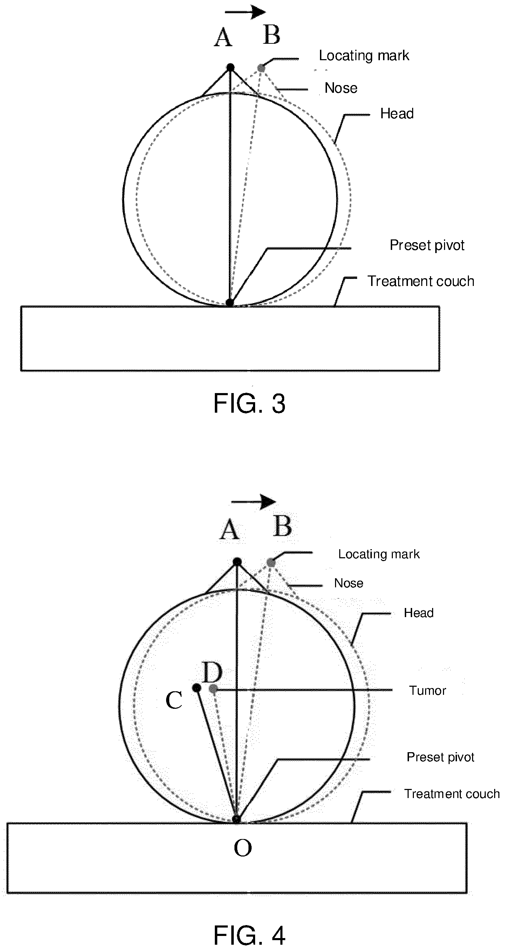

Specifically, considering that in the case where the position of the tumor coincides with the preset pivot, when the patient lies in the treatment couch, and the patient's head is fixed by the positioning mask on the preset pivot of the treatment couch, there is no relative movement between the patient's head and the preset pivot when the patient's head is rotated around the preset pivot. Therefore, the position of the tumor does not move with the movement of the patient's head. FIG. 3 is a schematic diagram showing movement of the patient's head. The solid line portion in FIG. 3 is the position of the patient's head before the movement, and the dotted line portion is the position of the patient's head after the movement. It can be seen that the position of the tumor does not move with the movement of the patient's head when the position of the tumor coincides with the preset pivot. Therefore, in the embodiments of the present invention, after determining that the position of the tumor coincides with the preset pivot, it can be known that the position of the tumor after the patient's head moves is still the position of the tumor before the patient's head moves.

In S102b-3, in a case where it is determined that the position of the tumor before the patient's head moves does not coincide with the preset pivot, the position of the tumor after the patient's head moves is determined according to the position of the positioning mark before the patient's head moves, the position of the positioning mark after the patient's head moves, and the position of the tumor before the patient's head moves.

Specifically, in one implementation manner, S101 includes: obtaining a coordinate value$(X_A, Y_A, Z_A)$ of the coordinate point A of the first positioning mark in the space coordinate system before the patient's head moves, and a coordinate value $(X_B, Y_B, Z_B)$ of the coordinate point B of the first positioning mark in the space coordinate system after the patient's head moves, and a coordinate value $(X_C, Y_C, Z_C)$ of the coordinate point C of the tumor in the space coordinate system before the patient's head moves.

The space coordinate system is a coordinate system established relative to the treatment couch, and the preset pivot is the origin O of the spatial coordinate system.

For example, FIG. 4 is a schematic diagram showing movement of the patient's head. The solid line portion in FIG. 4 is the position of the patient's head before the movement, and the dotted line portion is the position of the patient's head after the movement. The point A is the position of the first positioning mark before the patient's head moves, the point B is the position of the first positioning mark after the patient's head moves; the point C is the position of the tumor before the patient's head moves, and point D is the position of the tumor after the patient's head moves. In the exemplary figure, the first positioning mark is attached to the nasal tip of the head. The point O is the preset pivot.

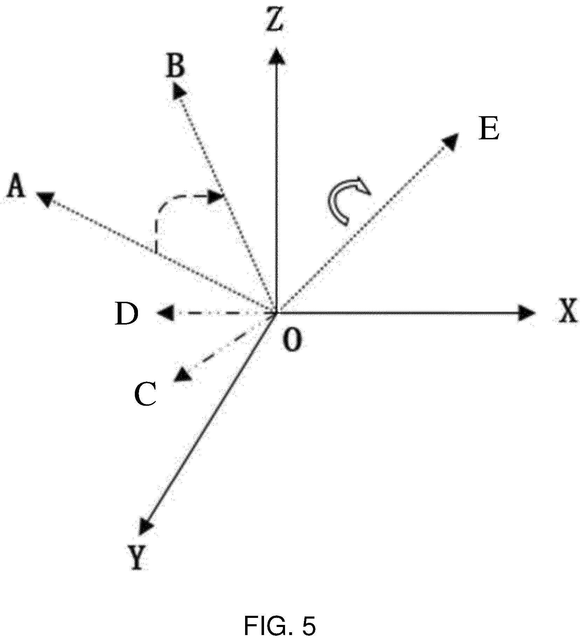

Further, as shown in FIG. 5, a coordinate system corresponding to a treatment couch is established at a preset pivot as an origin O of a space coordinate system. A $(X_A, Y_A, Z_A)$ is the position of the first positioning mark before the head moves, and B $(X_B, Y_B, Z_B)$ is the position of the first positioning mark after the head moves. C $(X_C, Y_C, Z_C)$ is the position of the tumor before the head moves, and D $(X_D, Y_D, Z_D)$ is the position of the tumor after the head moves. Vector OE is a normal vector of vector OA and vector OB. The coordinates of A and B are detected by an optical tracking system to obtain the corresponding coordinate positions. The coordinate position of C is calculated from the relative relationship between the tumor and the pivot in the CT image. Therefore, as long as the D position is obtained, the motion of the tumor may be determined.

Further, S102b-3 may specifically include: calculating a coordinate value of the coordinate point D $(X_D, Y_D, Z_D)$ of the tumor in the space coordinate system after the patient's head moves through the following formula 1:

$$\text{formula 1：} \begin{bmatrix} X_D \\ Y_D \\ Z_D \\ 1 \end{bmatrix} = M * \begin{bmatrix} X_C \\ Y_C \\ Z_C \\ 1 \end{bmatrix};$$

M represents a rotation matrix M rotated by an angle $\alpha$ around the vector OE, the vector OE is the normal vector of the vector OA and the vector OB, the vector OA is a vector pointing from the origin O to the coordinate point A, the vector OB is a vector pointing from the origin O to the coordinate point B, and $\alpha$ is a degree of an angle $\angle$AB between the vector OA and the vector OB.

Further, the coordinate value of the coordinate point D of the tumor in the space coordinate system is calculated through formula 1 after the patient's head moves, which may include the following contents of steps (1)-(6).

(1) The vector OE is obtained by doing a cross-product of the vector OA and the vector OB, and the vector OE is:

$$(Y_A * Z_B - Z_A * Y_B, Z_A * X_B - X_A * Z_B, X_A * Y_B - Y_A * X_B).$$

(2) The degree of the angle $\angle$AB between the vector OA and the vector OB is:

$$\angle AB = a\cos\left(\frac{OA * OB}{|OA| * |OB|}\right).$$

(3) In the embodiments of the present invention, since there is only a rigid transformation in the human's head, and after the head is fixed on the treatment couch by the positioning mask, the head may only rotate around the preset pivot as the origin. According to the principle of three-dimensional space transformation, any rigid transformation may be converted to a fixed angle rotated around any axis, that is, the degree of $\angle$AB rotated around the vector OE in this embodiment.

Further, the method of rotating around any axis may be divided into:

    a. translating a rotation axis to coincide with the coordinate axis, a corresponding operation being T;
    b. rotating the degree of LAB, a corresponding operation being R;
    c. translating the rotation axis back to the original position, a corresponding operation being $T^{-1}$;

that is, a whole process being $P^t = P \cdot T \cdot R \cdot T^{-1}$.

(4) According to the rotation rule of the right-handed coordinate system, the rotation matrix in the three-dimensional space includes:

the rotation matrix when rotated around the X axis by $\beta$ degrees, being:

$$R_x(\beta) = \begin{bmatrix} 1 & 0 & 0 & 0 \\ 0 & \cos(\beta) & -\sin(\beta) & 0 \\ 0 & \sin(\beta) & \cos(\beta) & 0 \\ 0 & 0 & 0 & 1 \end{bmatrix};$$

the rotation matrix when rotated around the Y axis by $\beta$ degrees, being:

$$R_y(\beta) = \begin{bmatrix} \cos(\beta) & 0 & \sin(-\beta) & 0 \\ 0 & 1 & 0 & 0 \\ -\sin(-\beta) & 0 & \cos(\beta) & 0 \\ 0 & 0 & 0 & 1 \end{bmatrix};$$

the rotation matrix when rotated around the Z axis by β degrees, being:

$$R_z(\beta) = \begin{bmatrix} \cos(\beta) & \sin(\beta) & 0 & 0 \\ -\sin(\beta) & \cos(\beta) & 0 & 0 \\ 0 & 0 & 1 & 0 \\ 0 & 0 & 0 & 1 \end{bmatrix}.$$

(5) Based on the content of step (4), it can be known that, according to the rotation rule of the right-hand coordinate system, the rotation matrix M rotated around the vector by α degrees OE is:

$$M = R_x(\gamma) \bullet R_z(-\theta) \bullet R_y(\alpha) \bullet R_z(\theta) \bullet R_x(-\gamma)$$

$\gamma$ is an angle at which the vector OE is rotated around the X-axis to the XOY plane, and $\theta$ is an angle at which the OE vector that has been rotated to the XOY plane is rotated around Z-axis to coincide with the Y-axis, and $\alpha$ is an angle $\angle AB$ between the vector OA and the vector OB.

(6) Further, according to formula 1, that is:

$$\begin{bmatrix} X_D \\ Y_D \\ Z_D \\ 1 \end{bmatrix} = M * \begin{bmatrix} X_C \\ Y_C \\ Z_C \\ 1 \end{bmatrix}.$$

The Euclidean distance between point C and point D may be calculated and the motion situation of the tumor may be analyzed.

In the embodiments of the present invention, considering that when the patient's head is fixed on the treatment couch through the positioning mask, the patient's head will rotates around the preset pivot, but there is often no relative movement at the contact point between the patient's head and the treatment couch. As a result, it is possible to determine the positional change situation of the tumor by obtaining the positional change situation of one positioning mark. Further, by obtaining a coordinate value of the first positioning mark in the spatial coordinate system before and after the head moves, a rotation matrix M of the head is calculated, and then the rotation matrix M is used to calculate a coordinate value of the coordinate point D ($X_D$,$Y_D$,$Z_D$) of the tumor in the space coordinate system to achieve precisely positioning of the tumor.

Embodiment 2

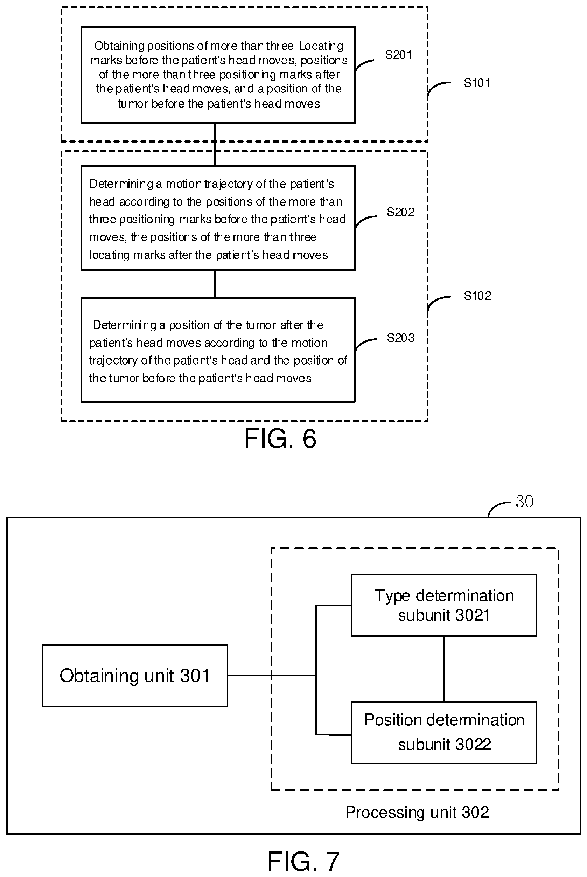

In one implementation manner, considering that a motion trajectory of the patient's head may be determined directly according to geometric transformation relationship in space of at least three positioning marks that are not on a same straight line, based on the method provided in the Embodiment 1, there are more than three positioning marks, and the more than three positioning marks are respectively disposed at different preset positions of the body surface of the patient's head, and the more than three positioning marks are not on a same straight line. As shown in FIG. 6, the method provided by the embodiments of the present invention may further specifically include the following steps.

S101 may specifically include the following step.

In S201, positions of more than three positioning marks before the patient's head moves, positions of the more than three positioning marks after the patient's head moves, and a position of the tumor before the patient's head moves are obtained.

The more than three positioning marks are disposed at different preset positions of the body surface of the patient's head, and the more than three positioning marks are not on the same straight line.

S102 may specifically include S202-S203.

In S202, a movement trajectory of the patient's head is determined according to the positions of the more than three positioning marks before the patient's head moves, the positions of the more than three positioning marks after the patient's head moves.

In S203, a position of the tumor after the patient's head moves is determined according to the motion trajectory of the patient's head and the position of the tumor before the patient's head moves.

Embodiment 3

The embodiments of the present invention further provide an apparatus of positioning a tumor based on the method of positioning a tumor provided by the above embodiments. As shown in FIG. 7, the apparatus 30 of positioning a tumor includes:

an obtaining unit 301 used to obtain a position of a positioning mark before the patient's head moves, a position of the positioning mark after the patient's head moves, and a position of the tumor before the patient's head moves, the positioning mark being disposed at a preset position of the body surface of the patient's head;

a processing unit 302 used to determine a position of the tumor after the patient's head moves according to the position of the positioning mark before the patient's head moves, the position of the positioning mark after the patient's head moves, and the position of the tumor before the patient's head moves, after the obtaining unit obtains the position of the positioning mark before the patient's head moves, the position of the positioning mark after the patient's head moves, and the position of the tumor before the patient's head moves.

Optionally, as shown in FIG. 7, the processing unit 302 specifically includes: a type determination subunit 3021 and a position determination subunit 3022.

The type determination subunit 3021 is used to determine a motion type of the patient's head according to the position of the positioning mark before the patient's head moves, and the position of the positioning mark after the patient's head moves. The motion type at least includes one or two of translation and rotation around a preset pivot.

The position determination subunit 3022 is used to determine the position of the tumor after the patient's head moves according to the motion type of the patient's head and the position of the tumor before the patient's head moves.

Optionally, the type determination subunit 3021 is specifically used to determine an amount of movement of the positioning mark according to the position of the positioning mark before the patient's head moves, and the position of the positioning mark after the patient's head moves; and to determine the motion type of the patient's head according to the amount of movement of the positioning mark.

Optionally, the position determination subunit 3022 is used to determine the position of the tumor after the patient's head moves according to the amount of movement of the positioning mark and the position of the tumor before the patient's head moves, in a case where it is determined that the motion type of the patient's head is the translation.

Optionally, the position determination subunit 3022 is specifically configured to determine whether the position of the tumor before the patient's head moves coincides with the preset pivot, in a case where it is determined that the motion type of the patient's head is the rotation around the preset pivot; in a case where it is determined that the position of the tumor before the patient's head moves coincides with the preset pivot, the position of the tumor after the patient's head moves is still the position of the tumor before the patient's head moves; and in a case where it is determined that the position of the tumor before the patient's head moves does not coincide with the preset pivot, the position of the tumor after the patient's head moves is determined according to the position of the positioning mark before the patient's head moves, the position of the positioning mark after the patient's head moves, and the position of the tumor before the patient's head moves.

Optionally, there are more than three positioning marks, and the more than three positioning marks are disposed at different preset positions of the body surface of the patient's head, and the more than three positioning marks are not on a same straight line.

Since the apparatus of positioning a tumor in the embodiments of the present invention may be applied to implement the above-mentioned method embodiments, the technical effects that may be obtained can also be referred to the foregoing method embodiments, which will not be described in the embodiment again.

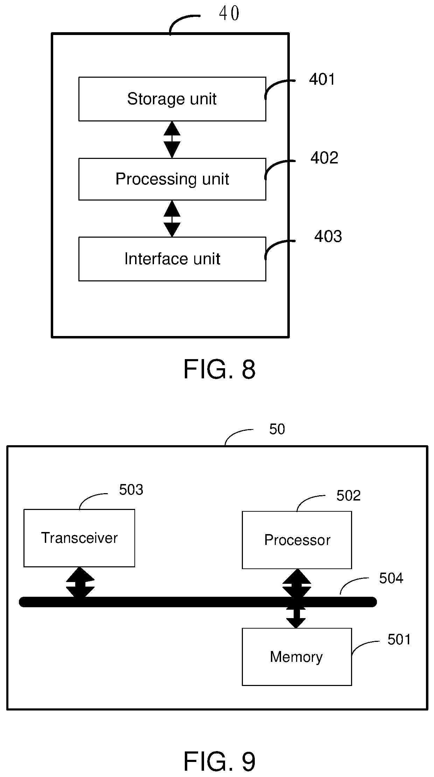

In a case where an integrated unit is adopted, FIG. 8 shows a possible schematic diagram showing a structure of the above apparatus of positioning a tumor. The apparatus 40 of positioning a tumor includes a storage unit 401, a processing unit 402, and an interface unit 403. The processing unit 402 is used to control and managing actions of the apparatus 40 of positioning a tumor.

For example, the processing unit is a processor, the storage unit is a memory, and the interface unit is a transceiver. Further, the apparatus of positioning a tumor 50 shown in FIG. 9 includes a transceiver 503, a processor 502, a memory 501, and a bus 504. The transceiver 503 and the processor 502 are connected to the memory 501 via the bus 504.

The processor 502 may be a general purpose central processing unit (CPU), a microprocessor, an application-specific integrated circuit (ASIC), or one or more integrated circuits to control the execution of the program of the present invention.

The memory 501 may be a read-only memory (ROM) or any other type of static storage device that can store static information and instructions, a random access memory (RAM) or any other type of dynamic storage device that can store information and instructions. The memory 501 may also be an electrically erasable programmable read-only memory (EEPROM), a compact disc read-only memory (CD-ROM), or any other optical disc storage, disc storage (including compact discs, laser discs, optical discs, digital versatile discs, blu-ray discs, etc.), a disk storage medium or any other magnetic storage device, or any other medium that can be used to carry or store desired program code in a form of instructions or data structures and can be accessed by a computer, which is not limited thereto. The memory may exist independently and be connected to the processor via a bus. The memory may also be integrated with the processor.

The memory 501 is used to store application program codes for executing the solutions of the present invention, which is controlled by the processor 502 for execution. The transceiver 503 is configured to receive the content input by an external device, and the processor 502 is configured to execute the application program codes stored in the memory 501, thereby implementing the method of positioning the tumor described in the embodiments of the present invention.

The above embodiments may be implemented in whole or in part through software, hardware, firmware, or any combination thereof. When implemented by using a software program, the embodiments may be implemented in whole or in part in the form of a computer program product. The computer program product includes one or more computer instructions. When the computer program instructions are loaded and executed on the computer, the processes or functions described are generated in whole or in part in accordance with embodiments of the present invention. The computer can be a general purpose computer, a special purpose computer, a computer network, or any other programmable device. The computer instructions can be stored in a computer readable storage medium or transferred from one computer readable storage medium to another computer readable storage medium, for example, the computer instructions can be transferred from a website site, computer, server or data center to another website site, computer, server, or data center by means of wire (e.g., coaxial cable, fiber optic, digital subscriber line (DSL)) or wireless (e.g., infrared, wireless, microwave, etc.). The computer readable storage medium may be any available medium that can be accessed by the computer or a data storage device that includes one or more servers, data centers, etc. that can be integrated with a medium. The usable medium may be a magnetic medium (e.g., a floppy disk, a hard disk, a magnetic tape), an optical medium (e.g., a DVD), or a semiconductor medium (such as a solid state disk (SSD)), or the like.

The embodiments of the present invention further provide a computer storage medium, which includes instructions, and when the instructions are run on a computer, the computer is caused to perform the method of positioning a tumor as provided in the above embodiments.

The embodiments of the present invention further provide a computer program product including instructions, and when the instructions are run on a computer, the computer is caused to perform the method of positioning a tumor as provided in the above embodiments.

The invention is defined in the following claims. Other embodiments, items, examples, methods etc. are not a part of the invention.

## Claims

1. A method of positioning a tumor, the method being applied to positioning a tumor in a patient's head, and the method comprising:

    obtaining a position of a positioning mark before the patient's head moves, a position of the positioning mark after the patient's head moves, and a position of the tumor before the patient's head moves, wherein the positioning mark is disposed at a preset position of a body surface of the patient's head; and
    determining a position of the tumor after the patient's head moves according to the position of the positioning mark before the patient's head moves, the position of the positioning mark after the patient's head moves, and the position of the tumor before the patient's head moves,
    wherein determining the position of the tumor after the patient's head moves according to the position of the positioning mark before the patient's head moves, the position of the positioning mark after the patient's head moves, and the position of the tumor before the patient's head moves, includes:
    determining a motion type of the patient's head according to the position of the positioning mark before the patient's head moves, and the position of the positioning mark after the patient's head moves, wherein the motion type at least includes one or two of translation and rotation around a preset pivot; and

determining the position of the tumor after the patient's head moves according to the motion type of the patient's head and the position of the tumor before the patient's head moves.

2. The method of positioning a tumor according to claim 1, **characterized in that** determining the motion type of the patient's head according to the position of the positioning mark before the patient's head moves, and the position of the positioning mark after the patient's head moves, includes:

   determining an amount of movement of the positioning mark according to the position of the positioning mark before the patient's head moves, and the position of the positioning mark after the patient's head moves; and
   determining the motion type of the patient's head according to the amount of movement of the positioning mark.

3. The method of positioning a tumor according to claim 1, wherein in a case where it is determined that the motion type of the patient's head is the translation, determining the position of the tumor after the patient's head moves according to the motion type of the patient's head, and the position of the tumor before the patient's head moves, includes: determining the position of the tumor after the patient's head moves according to an amount of movement of the positioning mark and the position of the tumor before the patient's head moves.

4. The method of positioning a tumor according to claim 1, wherein in a case where it is determined that the motion type of the patient's head is the rotation around the preset pivot, determining the position of the tumor after the patient's head moves according to the motion type of the patient's head, and the position of the tumor before the patient's head moves, includes:

   determining whether the position of the tumor before the patient's head moves coincides with the preset pivot;
   in a case where it is determined that the position of the tumor before the patient's head moves coincides with the preset pivot, the position of the tumor after the patient's head moves is still the position of the tumor before the patient's head moves; and
   in a case where it is determined that the position of the tumor before the patient's head moves does not coincide with the preset pivot, determining the position of the tumor after the patient's head moves according to the position of the positioning mark before the movement of the patient's head, the position of the positioning mark after the movement of the patient's head, and the position of the tumor before the movement of the patient's head.

5. The method of positioning a tumor according to claim 1, wherein there are more than three positioning marks, and the more than three positioning marks are respectively disposed at different preset positions of the body surface of the patient's head, and the more than three positioning marks are not on a same straight line.

6. An apparatus of positioning a tumor, wherein the apparatus (50) comprises: a processor (502), a memory (501), a bus (504) and a communication interface; the memory (501) is used to store computer execution instructions, and the processor (502) is connected to the memory (501) through the bus (504), and when a cache server is running, the processor (502) executes the computer execution instructions stored by the memory (501) to cause the apparatus of positioning the tumor to perform the method of positioning the tumor according to any one of claims 1 to 5.

7. A computer storage medium comprising instructions, and when the instructions are run on a computer, the computer is caused to perform the method of positioning the tumor according to any one of claims 1 to 5.

8. A computer program product including instructions, wherein when the instructions are run on a computer, the computer is caused to perform the method of positioning the tumor according to any one of claims 1 to 5.

**Patentansprüche**

1. Verfahren zur Positionierung eines Tumors, wobei das Verfahren auf die Positionierung eines Tumors in dem Kopf eines Patienten angewendet wird, und das Verfahren umfassend:

   Erhalten einer Position eines Positionierungszeichens, bevor sich der Kopf des Patienten bewegt, einer Position des Positionierungszeichens, nachdem sich der Kopf des Patienten bewegt hat, und einer Position des Tumors, bevor sich der Kopf des Patienten bewegt, wobei das Positionierungszeichen an einer voreingestellten Position auf der Körperoberfläche des Kopfes des Patienten angeordnet ist; und
   Bestimmen einer Position des Tumors, nachdem sich der Kopf des Patienten bewegt hat, nach der Position des Positionierungszeichens, bevor sich der Kopf des Patienten bewegt, der Position des Positionierungszeichens, nachdem sich der Kopf des Patienten bewegt hat, und der Position des Tumors, bevor sich der Kopf des Patienten bewegt,

wobei Bestimmen der Position des Tumors, nachdem sich der Kopf des Patienten bewegt hat, nach der Position des Positionierungszeichens, bevor sich der Kopf des Patienten bewegt, der Position des Positionierungszeichens, nachdem sich der Kopf des Patienten bewegt hat, und der Position des Tumors, bevor sich der Kopf des Patienten bewegt umfasst:

Bestimmen eines Bewegungstyps des Kopfes des Patienten nach der Position des Positionierungszeichens, bevor sich der Kopf des Patienten bewegt, und der Position des Positionierungszeichens, nachdem sich der Kopf des Patienten bewegt hat, wobei der Bewegungstyp mindestens eine oder beide zwischen Verschiebung und Drehung um ein voreingestellten Drehzentrum umfasst; und

Bestimmen der Position des Tumors, nachdem sich der Kopf des Patienten bewegt hat, nach dem Bewegungstyp des Kopfes des Patienten und der Position des Tumors, bevor sich der Kopf des Patienten bewegt.

2.  Verfahren zur Positionierung eines Tumors nach Anspruch 1, **dadurch gekennzeichnet, dass** Bestimmen des Bewegungstyps des Kopfes des Patienten nach der Position des Positionierungszeichens, bevor sich der Kopf des Patienten bewegt, und der Position des Positionierungszeichens, nachdem sich der Kopf des Patienten bewegt hat, umfasst:

Bestimmen eines Bewegungsniveaus des Positionierungszeichens nach der Position des Positionierungszeichens, bevor sich der Kopf des Patienten bewegt, und der Position des Positionierungszeichens, nachdem sich der Kopf des Patienten bewegt hat; und

Bestimmen des Bewegungstyps des Kopfes des Patienten nach dem Bewegungsniveau des Positionierungszeichens.

3.  Verfahren zur Positionierung eines Tumors nach Anspruch 1, wobei in einem Fall, wenn es bestimmt wird, dass der Bewegungstyp des Kopfes des Patienten eine Verschiebung ist, Bestimmen der Position des Tumors, nachdem sich der Kopf des Patienten bewegt hat, nach dem Bewegungstyp des Kopfes des Patienten und der Position des Tumors, bevor sich der Kopf des Patienten bewegt, umfasst:
    Bestimmen der Position des Tumors, nachdem sich der Kopf des Patienten bewegt hat, nach einem Bewegungsniveau des Positionierungszeichens und der Position des Tumors, bevor sich der Kopf des Patienten bewegt.

4.  Verfahren zur Positionierung eines Tumors nach Anspruch 1, wobei in einem Fall, wenn es bestimmt wird, dass der Bewegungstyp des Kopfes des Patienten eine Drehung um das voreingestellte Drehzentrum ist, Bestimmen der Position des Tumors, nachdem sich der Kopf des Patienten bewegt hat, nach dem Bewegungstyp des Kopfes des Patienten und der Position des Tumors, bevor sich der Kopf des Patienten bewegt, umfasst:

Bestimmen, ob die Position des Tumors, bevor sich der Kopf des Patienten bewegt, mit dem voreingestellten Drehzentrum übereinstimmt;

wobei in einem Fall, wenn es bestimmt wird, dass die Position des Tumors, bevor sich der Kopf des Patienten bewegt, mit dem voreingestellten Drehzentrum übereinstimmt, die Position des Tumors, nachdem sich der Kopf des Patienten bewegt hat, noch die Position des Tumors ist, bevor sich der Kopf des Patienten bewegt; und

in einem Fall, wenn es bestimmt wird, dass die Position des Tumors, bevor sich der Kopf des Patienten bewegt, nicht mit dem voreingestellten Drehzentrum übereinstimmt, Bestimmen der Position des Tumors, nachdem sich der Kopf des Patienten bewegt hat, nach der Position des Positionierungszeichens vor der Bewegung des Kopfes des Patienten, der Position des Positionierungszeichens nach der Bewegung des Kopfes des Patienten und der Position des Tumors vor der Bewegung des Kopfes des Patienten.

5.  Verfahren zur Positionierung eines Tumors nach Anspruch 1, wobei mehr als drei Positionierungszeichen vorhanden sind, und die mehr als drei Positionierungszeichen jeweils an unterschiedlichen voreingestellten Positionen der Körperoberfläche des Kopfes des Patienten angeordnet sind, und die mehr als drei Positionierungszeichen nicht auf einer gemeinsamen Geraden liegen.

6.  Gerät zur Positionierung eines Tumors, wobei das Gerät (50) umfasst: einen Prozessor (502), einen Speicher (501), einen Bus (504) und eine Kommunikationsschnittstelle; wobei der Speicher (501) verwendet wird, um computerausführbaren Anweisungen zu speichern, und der Prozessor (502) über den Bus (504) mit dem Speicher (501) verbunden ist, und wenn ein Cache-Server läuft, führt der Prozessor (502) die computerausführbaren Anweisungen aus, die durch den Speicher (50) gespeichert werden, um das Gerät zur Positionierung des Tumors zu veranlassen, das Verfahren zur Positionierung des Tumors nach einem der Ansprüche 1 bis 5 auszuführen.

7. Computer-Speichermedium umfassend Anweisungen, und wenn die Anweisungen auf einem Computer ausgeführt werden, wird der Computer veranlasst, das Verfahren zur Positionierung des Tumors nach einem der Ansprüche 1 bis 5 auszuführen.

8. Computerprogrammprodukt umfassend Anweisungen, wobei, wenn die Anweisungen auf einem Computer ausgeführt werden, der Computer veranlasst wird, das Verfahren zur Positionierung des Tumors nach einem der Ansprüche 1 bis 5 auszuführen.

**Revendications**

1. Procédé de positionnement d'une tumeur, le procédé étant appliqué au positionnement d'une tumeur dans la tête d'un patient, et le procédé comprenant :

  obtenir une position d'une marque de positionnement avant que la tête du patient ne se déplace, une position de la marque de positionnement après que la tête du patient se soit déplacée, et une position de la tumeur avant que la tête du patient ne se déplace, la marque de positionnement étant disposée à une position prédéfinie d'une surface corporelle de la tête du patient ; et
  déterminer une position de la tumeur après que la tête du patient se soit déplacée en fonction de la position de la marque de positionnement avant que la tête du patient ne se déplace, de la position de la marque de positionnement après que la tête du patient se soit déplacée, et de la position de la tumeur avant que la tête du patient ne se déplace,
  la détermination de la position de la tumeur après que la tête du patient se soit déplacée en fonction de la position de la marque de positionnement avant que la tête du patient ne se déplace, de la position de la marque de positionnement après que la tête du patient se soit déplacée, et de la position de la tumeur avant que la tête du patient ne se déplace, comprenant :

  déterminer un type de mouvement de la tête du patient en fonction de la position de la marque de positionnement avant que la tête du patient ne se déplace, et de la position de la marque de positionnement après que la tête du patient se soit déplacée, le type de mouvement comprenant au moins l'un ou les deux éléments suivants : translation et rotation autour d'un pivot prédéfini ; et
  déterminer la position de la tumeur après que la tête du patient se soit déplacée en fonction du type de mouvement de la tête du patient et de la position de la tumeur avant que la tête du patient ne se déplace.

2. Le procédé de positionnement d'une tumeur selon la revendication 1, **caractérisé en ce que** la détermination du type de mouvement de la tête du patient en fonction de la position de la marque de positionnement avant que la tête du patient ne se déplace, et de la position de la marque de positionnement après que la tête du patient se soit déplacée, comprend :

  déterminer une amplitude de déplacement de la marque de positionnement en fonction de la position de la marque de positionnement avant que la tête du patient ne se déplace, et de la position de la marque de positionnement après que la tête du patient se soit déplacée ; et
  déterminer le type de mouvement de la tête du patient en fonction de l'amplitude de déplacement de la marque de positionnement.

3. Le procédé de positionnement d'une tumeur selon la revendication 1, dans le cas où il est déterminé que le type de mouvement de la tête du patient est une translation, la détermination de la position de la tumeur après que la tête du patient se soit déplacée en fonction du type de mouvement de la tête du patient, et de la position de la tumeur avant que la tête du patient ne se déplace, comprend :
déterminer la position de la tumeur après que la tête du patient se soit déplacée en fonction d'une amplitude de déplacement de la marque de positionnement et de la position de la tumeur avant que la tête du patient ne se déplace.

4. Le procédé de positionnement d'une tumeur selon la revendication 1, dans le cas où il est déterminé que le type de mouvement de la tête du patient est une rotation autour du pivot prédéfini, la détermination de la position de la tumeur après que la tête du patient se soit déplacée en fonction du type de mouvement de la tête du patient, et de la position de la tumeur avant que la tête du patient ne se déplace, comprend :

déterminer si la position de la tumeur avant que la tête du patient ne se déplace coïncide avec le pivot prédéfini ;
dans le cas où il est déterminé que la position de la tumeur avant que la tête du patient ne se déplace coïncide avec le pivot prédéfini, la position de la tumeur après que la tête du patient se soit déplacée est toujours la position de la tumeur avant que la tête du patient ne se déplace ; et
dans le cas où il est déterminé que la position de la tumeur avant que la tête du patient ne se déplace ne coïncide pas avec le pivot prédéfini, déterminer la position de la tumeur après que la tête du patient se soit déplacée en fonction de la position de la marque de positionnement avant le mouvement de la tête du patient, de la position de la marque de positionnement après le mouvement de la tête du patient, et de la position de la tumeur avant le mouvement de la tête du patient.

5. Le procédé de positionnement d'une tumeur selon la revendication 1, dans lequel il y a plus de trois marques de positionnement, et les plus de trois marques de positionnement sont respectivement disposées à différentes positions prédéfinies de la surface corporelle de la tête du patient, et les plus de trois marques de positionnement ne sont pas situées sur une même ligne droite.

6. Un dispositif de positionnement d'une tumeur, le dispositif (50) comprenant : un processeur (502), une mémoire (501), un bus (504) et une interface de communication ; la mémoire (501) est utilisée pour stocker des instructions exécutables par ordinateur, et le processeur (502) est connecté à la mémoire (501) par l'intermédiaire du bus (504), et lorsqu'un serveur cache fonctionne, le processeur (502) exécute les instructions exécutables par ordinateur stockées par la mémoire (501) pour amener le dispositif de positionnement de la tumeur à exécuter le procédé de positionnement de la tumeur selon l'une quelconque des revendications 1 à 5.

7. Un support de stockage informatique comprenant des instructions, et lorsque les instructions sont exécutées sur un ordinateur, l'ordinateur est amené à exécuter le procédé de positionnement de la tumeur selon l'une quelconque des revendications 1 à 5.

8. Un produit programme d'ordinateur comprenant des instructions, et lorsque les instructions sont exécutées sur un ordinateur, l'ordinateur est amené à exécuter le procédé de positionnement de la tumeur selon l'une quelconque des revendications 1 à 5.

FIG. 1

Obtaining a position of a locating mark before the patient's head moves, a position of the locating mark after the patient's head moves, and a position of the tumor before the patient's head moves
— S101

Determining a position of the tumor after the patient's head moves according to the position of the positioning mark before the patient's head moves, the position of the positioning mark after the patient's head moves, and the position of the tumor before the patient's head moves.
— S102

FIG. 2

FIG. 3

FIG. 4

FIG. 5

Obtaining positions of more than three Locating marks before the patient's head moves, positions of the more than three positioning marks after the patient's head moves, and a position of the tumor before the patient's head moves — S201

S101

Determining a motion trajectory of the patient's head according to the positions of the more than three positioning marks before the patient's head moves, the positions of the more than three locating marks after the patient's head moves — S202

S102

Determining a position of the tumor after the patient's head moves according to the motion trajectory of the patient's head and the position of the tumor before the patient's head moves — S203

## FIG. 6

30

Obtaining unit 301

Type determination subunit 3021

Position determination subunit 3022

Processing unit 302

## FIG. 7

40

Storage unit — 401

$\updownarrow$

Processing unit — 402

$\updownarrow$

Interface unit — 403

FIG. 8

50

Transceiver — 503

Processor — 502

— 504

Memory — 501

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102697561 A
- CN 106955122 A